# EUROPEAN PATENT SPECIFICATION

(11) **EP 3 624 717 B1**
(45) Date of publication and mention of the grant of the patent: **09.02.2022**
(21) Application number: 18760057.2
(22) Date of filing: 01.08.2018
(51) Int. Cl.: A61B 18/14, A61B 90/00, G01K 13/00, G01K 11/32, A61B 34/20, A61B 18/00, A61B 17/00, G01K 7/02, G01K 13/20, G01K 11/3206

(54) **OPTICAL FORCE SENSING CATHETER SYSTEM**
OPTISCHES KRAFTMESSENDES KATHETERSYSTEM
SYSTÈME CATHÉTER À DÉTECTION DE FORCE OPTIQUE

(30) Priority: 02.08.2017 US 201762540409 P
(43) Date of publication of application: 25.03.2020
(73) Proprietor: St. Jude Medical International Holding S.à r.l., 2449 Luxembourg (LU)
(72) Inventor: DALY, Jacob John, Ham Lake, MN 55304 (US); TEGG, Troy T., Elk River, MN 55330 (US)
(74) Representative: Kramer Barske Schmidtchen Patentanwälte PartG mbB
(86) International application number: PCT/IB2018/055799
(87) International publication number: WO 2019/026012

(56) References cited:
- WO-A1-2014/144312
- WO-A1-2016/196985
- US-A1- 2008 294 158
- US-A1- 2012 265 102
- US-A1- 2014 206 985
- US-A1- 2017 196 479
- US-B2- 8 567 265

## Description

### BACKGROUND

### a. Field

The instant disclosure relates generally to force sensing systems capable of determining a force applied at a distal tip of a medical catheter. More specifically, the disclosure relates to a force sensing system with a deformable body.

### b. Background Art

US2017/0196479 A1 discloses a medical device with a multi-core fiber for optical sensing. US 2008/0294158 A1 discloses an ablation catheter with flexible tip and methods of making the same.

Exploration and treatment of various organs (or vessels) is possible using catheter-based diagnostic and treatment systems. Catheters may be introduced through a vessel leading to an organ to be explored, or treated, or alternatively may be introduced directly through an incision made in a wall of the organ. Catheter-based surgical systems avoid the trauma and extended recuperation times typically associated with open surgical procedures.

To provide effective diagnosis and/or therapy, it is frequently necessary to first precisely map a zone to be treated. Mapping may be performed, for example, when it is desired to selectively ablate conductive pathways within a heart to treat a cardiac arrhythmia, such as atrial fibrillation. Often, the mapping procedure is complicated by difficulties in locating the zone(s) to be treated due to periodic movement of the heart throughout the cardiac cycle.

Catheter navigation and mapping systems often rely on manual catheter feedback and/or impedance measurements to determine when the catheter is properly positioned. These systems do not measure contact forces with the organ wall, or detect contact forces applied by the catheter against the organ wall that may modify the true wall location. Accordingly, the mapping of the organ may be inaccurate due to artifacts created by excessive contact forces.

To facilitate improved mapping, it is desirable to detect and monitor contact forces between a catheter tip and an organ wall to permit faster and more accurate mapping. Once the topography of the organ is mapped, either the same or a different catheter may be employed to effect treatment. Depending upon the specific treatment to be applied to the organ, the catheter may comprise any of a number of end effectors, such as, for example, RF ablation electrodes, mapping electrodes, etc.

The effectiveness of such end effectors often depends on the end effector contact with the wall tissue, which may be inherently unstable due to the motion of the organ (e.g., pumping motion of the cardiac muscle). Existing catheter-based force sensing systems often do not have the ability to accurately sense the load applied to the distal tip of the catheter associated with either movement of the catheter or the tissue wall in contact therewith. For example, in the case of a cardiac ablation system, at one extreme the creation of a gap between the end effector and the tissue wall may render the treatment ineffective, and inadequately ablate the tissue zone. At the other extreme, if the end effector of the catheter contacts the tissue wall with excessive force, it may inadvertently puncture the tissue.

In view of the foregoing, a catheter-based diagnostic or treatment system that permits sensing of the load applied to the distal extremity of the catheter, including periodic loads arising from movement of the organ, is desirable. It is further desirable to provide diagnostic and treatment apparatus that permit computation of forces applied to a distal tip of a catheter with reduced sensitivity to the location on the catheter tip at which the forces are applied.

The foregoing discussion is intended only to illustrate the present field and should not be taken as a disavowal of claim scope.

### BRIEF SUMMARY

Aspects of the present disclosure are directed toward systems and methods for detecting force applied to a distal tip of a medical catheter using a fiber-optic force sensor and processor circuitry. In particular, the instant disclosure relates to a deformable body near a distal tip of a medical catheter that deforms in response to a force applied at the distal tip. The fiber-optic force sensor detects various components of the deformation, and the processor circuitry, based on the detected components of the deformation, determines a force applied to the distal tip of the catheter.

Various embodiments of the present disclosure are directed to force-sensing catheter systems. One such system includes a catheter tip, a tip stem, a deformable body, and a manifold. The tip stem includes an inner and outer diameter, and an aperture that extends through a length of the tip stem. The outer diameter of the tip stem is coupled to the catheter tip. The deformable body is coupled to the outer diameter of the tip stem, and deforms in response to a force exerted on the catheter tip. The deformable body includes a lumen that extends along a longitudinal axis of the force-sensing catheter system. The manifold extends through the lumen of the deformable body and the inner diameter of the tip stem, and is coupled to an inner diameter of the tip stem and a proximal end of the deformable body. The manifold delivers irrigant to the distal tip. In more specific embodiments, the manifold may transmit a portion of the force exerted on the catheter tip, proximally, to the proximal end of the deformable body. Moreover, the manifold and deformable body may emulate a desired lateral-to-axial compliance ratio of the force-sensing catheter system by transmitting more or less of the force exerted on the catheter tip through the manifold.

Some embodiments of the present disclosure are directed to an ablation catheter tip assembly. The ablation catheter tip assembly includes an ablation catheter tip, a deformable body, and a manifold. The ablation catheter tip delivers energy to contacted tissue to induce necrosis of the contacted tissue. The deformable body is mechanically coupled to a proximal end of the ablation catheter tip, and deforms in response to a force exerted on the catheter tip. The deformable body may include a lumen that extends along a longitudinal axis of the force-sensing catheter system, through which the manifold extends. The manifold is mechanically coupled to the proximal end of the ablation catheter tip, and delivers irrigant to the ablation catheter tip. To limit deformation of the deformable body, in response to the force exerted on the catheter tip, the manifold may absorb a portion of the force. In more specific embodiments, the catheter tip assembly includes a thermocouple coupled near a distal end of the catheter tip and a wire or flexible electronic circuit communicatively coupled with the thermocouple. The wire or flexible electronic circuit extends proximally through the force-sensing catheter system and an aperture of the tip stem. The aperture of the tip stem facilitates hermetically sealing the aperture with the wire or flexible electronic circuit extending therethrough.

The foregoing and other aspects, features, details, utilities, and advantages of the present disclosure will be apparent from reading the following description and claims, and from reviewing the accompanying drawings. The invention is defined in claim 1, further preferred embodiments are defined in the dependent claims.

### BRIEF DESCRIPTION OF THE DRAWINGS

FIG. 1 is a diagrammatic overview of a system for force sensing, consistent with various embodiments of the present disclosure;
FIG. 1A is a block diagram of a force sensing system, consistent with various embodiments of the present disclosure;
FIG. 1B is a schematic depiction of an interferometric fiber optic sensor, consistent with various embodiments of the present disclosure;
FIG. 1C is a schematic depiction of a fiber Bragg grating optical strain sensor, consistent with various embodiments of the present disclosure;
FIG. 2A is an isometric side view of a partial ablation catheter tip assembly, consistent with various embodiments of the present disclosure;
FIG. 2B is a cross-sectional side view of the partial ablation catheter tip assembly of FIG. 2A, consistent with various embodiments of the present disclosure;
FIG. 2C is a cross-sectional, isometric back view of the partial ablation catheter tip assembly of FIG. 2A, consistent with various embodiments of the present disclosure;
FIG. 3A is a side view of a structural member of a fiber optic force sensing assembly, consistent with various embodiments of the present disclosure;
FIG. 3B is a front view of the structural member of FIG. 3A, consistent with various embodiments of the present disclosure;
FIG. 4A is an isometric side view of a partial ablation catheter tip assembly including a deformable body, consistent with various embodiments of the present disclosure;
FIG. 4B is a cross-sectional side view of the partial ablation catheter tip assembly of FIG. 4A, consistent with various embodiments of the present disclosure;
FIG. 4C is a back view of the partial ablation catheter tip assembly of FIG. 4A, consistent with various embodiments of the present disclosure;
FIG. 5 is a back view of an alternative, partial ablation catheter tip assembly, consistent with various embodiments of the present disclosure;
FIG. 6A is an isometric side view of a sensor coupler assembly, consistent with various embodiments of the present disclosure;
FIG. 6B is an isometric front view of the sensor coupler assembly of FIG. 6A, consistent with various embodiments of the present disclosure;
FIG. 7 is an isometric side view of a partial ablation catheter tip assembly including a coupler, consistent with various embodiments of the present disclosure;

While various embodiments discussed herein are amenable to modifications and alternative forms, aspects thereof have been shown by way of example in the drawings and will be described in further detail. It should be understood, however, that the intention is not to limit the disclosure to the particular embodiments described. On the contrary, the intention is to cover all modifications, equivalents, and alternatives falling within the scope of the disclosure including aspects defined in the claims.

### DETAILED DESCRIPTION OF EMBODIMENTS

Aspects of the present disclosure are directed toward systems and methods for detecting force applied to a distal tip of an intravascular medical catheter. In particular, the instant disclosure relates to a deformable body (also referred to as a structural member) near a distal tip of a medical catheter that deforms in response to a force applied at the distal tip. Force sensors, such as fiber-optic force sensors, detect various components of the deformation, and processor circuitry, based on the detected components of the deformation, determines a force applied to the distal tip of the catheter. Importantly, various aspects of the present disclosure are directed to withstanding high load forces exerted on the deformable body without plastically deforming.

Various embodiments of the present disclosure are directed to a deformable body for a catheter force sensing system. The force sensing system may be modular to facilitate application of the force sensing system on various types of catheters, and for various applications. Force sensing systems as disclosed herein may be calibrated to measure forces exerted on a distal tip of a medical catheter via fiber optic measurement of a cavity gap, for example. Such a force sensing system may be particularly useful for cardiovascular ablation catheters, where a distal tip of the catheter is positioned in contact with myocardial tissue that is to receive an ablation therapy and necrose in response to the treatment. Ablation therapy can be a useful treatment for patients with a cardiac arrhythmia (e.g., atrial fibrillation). The necrosed tissue facilitates electrical isolation of unwanted electrical impulses often emanating from pulmonary veins (and arrhythmic foci). By electrically isolating the foci from the left atrium of the cardiac muscle, for example, the symptoms of atrial fibrillation can be reduced or eliminated. To the extent that arrhythmic foci are located within a tissue ablation zone, the arrhythmic foci are destroyed.

In a typical ablation therapy for atrial fibrillation, pulmonary veins are treated in accordance to their likelihood of having arrhythmic foci. Often, all pulmonary veins are treated. A distal tip of the catheter may include electrophysiology electrodes (also referred to as spot electrodes) which help to expedite diagnosis and treatment of a source of a cardiac arrhythmia, and may also be used to confirm a successful ablation therapy by determining the isolation of the arrhythmic foci from the left atrium, for example, or the destruction of the arrhythmic foci entirely.

During an ablation therapy, a distal end of an ablation catheter tip contacts ablation targeted myocardial tissue in order to conductively transfer energy (e.g., radio-frequency, thermal, etc.) thereto. It has been discovered that consistent force, during a series of tissue ablations, forms a more uniform and transmural lesion line. Uniform lesion lines have been found to better isolate the electrical impulses produced by arrhythmic foci, thereby improving the overall efficacy of the ablation therapy. To achieve consistent force, aspects of the present disclosure utilize a deformable body in the ablation catheter tip. The deformable body deforms in response to forces being exerted upon a distal end of the ablation catheter tip. The deformation of the deformable body may then be measured by a measurement device (e.g., ultrasonic, magnetic, optical, interferometry, etc.). Based on the tuning of the deformable body and/or the calibration of the measurement device, the deformation can then be associated with a force exerted on the distal end of the ablation catheter tip (e.g., via a lookup table, formula(s), calibration matrix, etc.). The measurement device and/or processor circuitry may be used to determine the exerted force, and output a signal indicative of the force exerted on the catheter tip. The calculated force can then be displayed to a clinician or otherwise communicated. In some specific embodiments, the processor circuitry may intervene in the ablation therapy where the force exerted on the tissue by the catheter tip is too low or too high.

Aspects of the present disclosure are also directed to a deformable body for a force sensing system that facilitates the routing of wires, thermocouples, irrigation lumens, and flexible circuitry, for example, through an inner diameter of the deformable body to a distal tip of the catheter. The ability to extend thermocouples distal of the deformable body is particularly advantageous for ablation catheter applications as the temperature readings from the thermocouples will be far more accurate and instantaneous. Further, in some embodiments, the deformable body may also be stiffened to withstand high load forces. During insertion of the catheter through an introducer, and/or while traveling through the vasculature of a patient, the distal tip of the catheter (and therefore the deformable body by virtue of the mechanical coupling of the two components) may experience large forces. In some applications, the deformable body may experience forces up to 1,000 grams. In various embodiments, a pivot point of a flexure portion of the deformable body is extended radially outwards to facilitate stiffening of the deformable body to withstand large forces without plastically deforming. This also facilitates a larger inner diameter for routing wires and other components through the deformable body. Plastic deformation is particularly problematic for catheter-based force sensing applications as the new set of the deformable body renders the factory calibration of the force sensing system inaccurate. Due to such plastic deformation, when the catheter is in a non-contact position with the cardiovascular system of a patient, the force sensing system may return a force indicative of contact between the distal tip and tissue. To prevent such plastic deformation, pivot points of flexure portions within the deformable body may be radially extended, increasing the stiffness and limiting total deflection to less than 3,000 nanometers. Yet further embodiments of the deformable body may extend an outer diameter to decrease the effect of a bending moment applied along a longitudinal axis of the deformable body. This outer diameter further increases the stiffness of the deformable body. Moreover, by extending the outer diameter the fulcrum arm created by the flexure portions will deflect a greater distance for the same lateral deflection of the deformable body facilitating improved measurement resolution of the measurement device.

The deformable body disclosed herein may further be manufactured on a modular platform which facilitates the use of a single force sensor assembly on a number of different medical catheters.

Details of the various embodiments of the present disclosure are described below with specific reference to the figures.

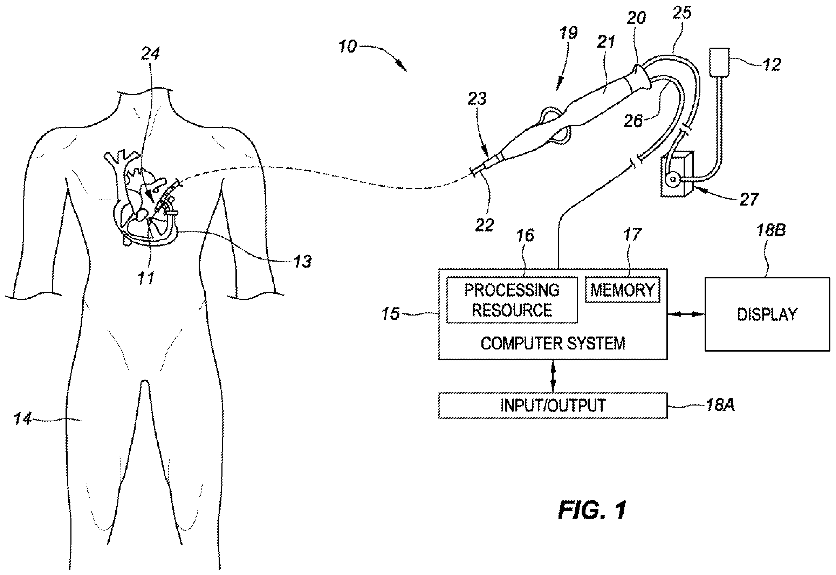

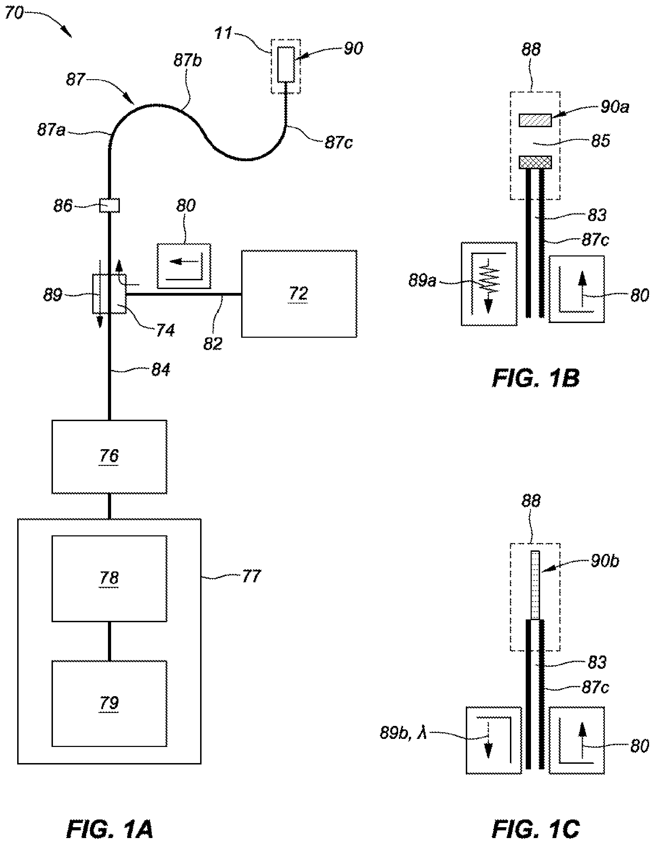

Referring now to the drawings wherein like reference numerals are used to identify identical components in the various views, Figure 1 generally illustrates a system 10 for force detection having an elongated medical device 19. The medical device includes a fiber optic force sensor assembly 11 configured to be used in the body for medical procedures. The fiber optic force sensor assembly 11 is included as part of a medical device such as an elongated medical device 19 and may be used for diagnosis, visualization, and/or treatment of tissue 13 (such as cardiac or other tissue) in the body. For example, the fiber optic force sensor assembly 11 may be used for ablation therapy of tissue 13 or mapping purposes in a patient's body 14. Figure 1 further shows various sub-systems included in the overall system 10. The system 10 may include a main computer system 15 (including an electronic control unit 16 and data storage 17, e.g., memory). The computer system 15 may further include conventional interface components, such as various user input/output mechanisms 18A and a display 18B, among other components. Information provided by the fiber optic force sensor assembly 11 may be processed by the computer system 15 and may provide data to the clinician via the input/output mechanisms 18A and/or the display 18B, or in other ways as described herein. Specifically, the display 18B may visually communicate a force exerted on the elongated medical device 19 - where the force exerted on the elongated medical device 19 is detected in the form of a deformation of at least a portion of the elongated medical device by the fiber optic force sensor assembly 11, and the measured deformations are processed by the computer system 15 to determine the force exerted.

In the illustrative embodiment of FIG. 1, the elongated medical device 19 may include a cable connector or interface 20, a handle 21, a tubular body or shaft 22 having a proximal end 23 and a distal end 24. The elongated medical device 19 may also include other conventional components not illustrated herein, such as a temperature sensor, additional electrodes, and corresponding conductors or leads. The connector 20 may provide mechanical, fluid and/or electrical connections for cables 25, 26 extending from a fluid reservoir 12 and a pump 27 and the computer system 15, respectively. The connector 20 may comprise conventional components known in the art and, as shown, may be disposed at the proximal end of the elongated medical device 19.

The handle 21 provides a portion for a user to grasp or hold the elongated medical device 19 and may further provide a mechanism for steering or guiding the shaft 22 within the patient's body 14. For example, the handle 21 may include a mechanism configured to change the tension on a pull-wire extending through the elongated medical device 19 to the distal end 24 of the shaft 22 or some other mechanism to steer the shaft 22. The handle 21 may be conventional in the art, and it will be understood that the configuration of the handle 21 may vary. In an embodiment, the handle 21 may be configured to provide visual, auditory, tactile and/or other feedback to a user based on information received from the fiber optic force sensor assembly 11. For example, if contact to tissue 13 is made by distal tip 24, the fiber optic force sensor assembly 11 will transmit data to the computer system 15 indicative of the contact. In response to the computer system 15 determining that the data received from the fiber optic force sensor assembly 11 is indicative of a contact between the distal tip 24 and a patient's body 14, the computer system 15 may operate a light-emitting-diode on the handle 21, a tone generator, a vibrating mechanical transducer, and/or other indicator(s), the outputs of which could vary in proportion to the signal sensed by the fiber optic force sensor assembly 11.

The computer system 15 can utilize software, hardware, firmware, and/or logic to perform a number of functions described herein. The computer system 15 can be a combination of hardware and instructions to share information. The hardware, for example can include processing resource 16 and/or a memory 17 (e.g., non-transitory computer-readable medium (CRM) database, etc.). A processing resource 16, as used herein, can include a number of processors capable of executing instructions stored by the memory resource 17. Processing resource 16 can be integrated in a single device or distributed across multiple devices. The instructions (e.g., computer-readable instructions (CRI)) can include instructions stored on the memory 17 and executable by the processing resource 16 for force detection.

The memory resource 17 can be in communication with the processing resource 16. A memory 17, as used herein, can include a number of memory components capable of storing instructions that can be executed by processing resource 16. Such a memory 17 can be a non-transitory computer readable storage medium, for example. The memory 17 can be integrated in a single device or distributed across multiple devices. Further, the memory 17 can be fully or partially integrated in the same device as the processing resource 16 or it can be separate but accessible to that device and the processing resource 16. Thus, it is noted that the computer system 15 can be implemented on a user device and/or a collection of user devices, on a mobile device and/or a collection of mobile devices, and/or on a combination of the user devices and the mobile devices.

The memory 17 can be in communication with the processing resource 16 via a communication link (e.g., path). The communication link can be local or remote to a computing device associated with the processing resource 16. Examples of a local communication link can include an electronic bus internal to a computing device where the memory 17 is one of a volatile, non-volatile, fixed, and/or removable storage medium in communication with the processing resource 16 via the electronic bus.

In various embodiments of the present disclosure, the computer system 15 may receive optical signals from a fiber optic force sensor assembly 11 via one or more optical fibers extending a length of the catheter shaft 22. A processing resource 16 of the computer system 15 may execute an algorithm stored in memory 17 to compute a force exerted on catheter tip 24, based on the received optical signals.

United States Patent no. 8,567,265 discloses various optical force sensors for use in medical catheter applications.

FIG. 1A is a block diagram of a force sensing system 70, consistent with various embodiments of the present disclosure. The force sensing system 70 may comprise an electromagnetic source 72, a coupler 74, a receiver 76, an operator console 77 operatively coupled with a microprocessor 78 and a storage device 79. The electromagnetic source 72 transmits electromagnetic radiation 80 (photons) that is substantially steady state in nature, such as a laser or a broadband light source. A transmission line 82 such as a fiber optic cable carries the radiation 80 to the coupler 74, which directs the radiation 80 through a transmitting/receiving line 84 and through a fiber optic element contained within a flexible, elongated catheter assembly 87 to a fiber optic force sensing element 90 within a fiber optic force sensor assembly 11. It is to be understood that while various embodiments of the present disclosure are directed to force sensing systems with fiber optic force sensing elements for detecting a change in dimension (e.g., deformation) of a catheter assembly 87, various other embodiments may include non-fiber optic based measurement systems as are well known in the art. Moreover, it is to be understood that the force sensing elements (also referred to as sensing elements) measure the deformation of a deformable body (e.g., a distance or displacement), and do not directly measure a force. The catheter assembly 87 may include one or more transmitting/receiving lines 84 coupled to one or more fiber optic elements 83 (as shown in FIGs. 1B-C) within the fiber optic force sensor assembly 11. The fiber optic element(s) 83 of the catheter assembly 87 and transmitting/receiving(s) line 84 may be coupled through a connector 86 as depicted in FIG. 1A.

The catheter assembly 87 may have a width and a length suitable for insertion into a bodily vessel or organ. In one embodiment, the catheter assembly 87 comprises a proximal portion 87a, a middle portion 87b and a distal portion 87c. The distal portion 87c may include an end effector which may house the fiber optic force sensor assembly 11 and the one or more fiber optic force sensing element(s) 90. The catheter assembly may be of a hollow construction (i.e. having a lumen) or of a non-hollow construction (i.e. no lumen), depending on the application.

In response to a deformation of a deformable body, due to a force being exerted on a distal tip of a catheter, one or more fiber optic elements 83 (as shown in FIGs. 1B-C) within the fiber optic force sensor assembly 11 will modulate the radiation received from the transmission line 82 and transmit the modulated radiation 89 to the operator console 77 via receiving line 84. Once the radiation is received by the operator console 77, a microprocessor 78 may run an algorithm stored on storage device 79 to determine a distance across the force sensing element(s) 90 and associate the distance with a force exerted on the catheter tip.

A fiber optic force sensing element 90, for detecting a deformation of a deformable body, may be an interferometric fiber optic strain sensor, a fiber Bragg grating strain sensor, or other fiber optic sensor well known in the art.

Referring to FIG. 1B, fiber optic force sensing element 88 is an interferometric fiber optic strain sensor 90a. In this embodiment, the transmitted radiation 80 enters an interferometric gap 85 within the interferometric fiber optic strain sensor 90a. A portion of the radiation that enters the interferometric gap 85 is returned to a distal portion 87c of catheter assembly 87 as a modulated waveform 89a. The various components of the interferometric fiber optic strain sensor 90a may comprise a structure that is integral with the fiber optic element 83. Alternatively, the fiber optic element 83 may cooperate with the structure to which it is mounted to form the interferometric gap 85.

Referring to FIG. 1C, fiber optic force sensing element 88, of FIG. 1A, is a fiber Bragg grating strain sensor 90b. In this embodiment, the transmitted radiation 80 enters a fiber Bragg grating 90b, the gratings of which are typically integral with the fiber optic element 83 and reflect only a portion 89b of the transmitted radiation 80 about a central wavelength λ. The central wavelength λ at which the portion 89b is reflected is a function of the spacing between the gratings of the fiber Bragg grating. Therefore, the central wavelength λ is indicative of the strain on the fiber Bragg grating strain sensor 90b relative to some reference state.

The reflected radiation 89, be it the modulated waveform 89a (as in FIG. 1B) or the reflected portion 89b (as in FIG. 1C), is transmitted back through the transmitting/receiving line 84 to the receiver 76. The strain sensing system 70 may interrogate the one or more fiber optic strain sensing element(s) 90 at an exemplary and non-limiting rate of 10-Hz. The receiver 76 is selected to correspond with the type of strain sensing element 90 utilized. That is, the receiver may be selected to either detect the frequency of the modulated waveform 89a for use with the interferometric fiber optic strain sensor 90a, or to resolve the central wavelength of the reflected portion 89b for use with fiber Bragg grating strain sensor 90b. The receiver 76 manipulates and/or converts the incoming reflected radiation 89 into digital signals for processing by the microprocessor 78.

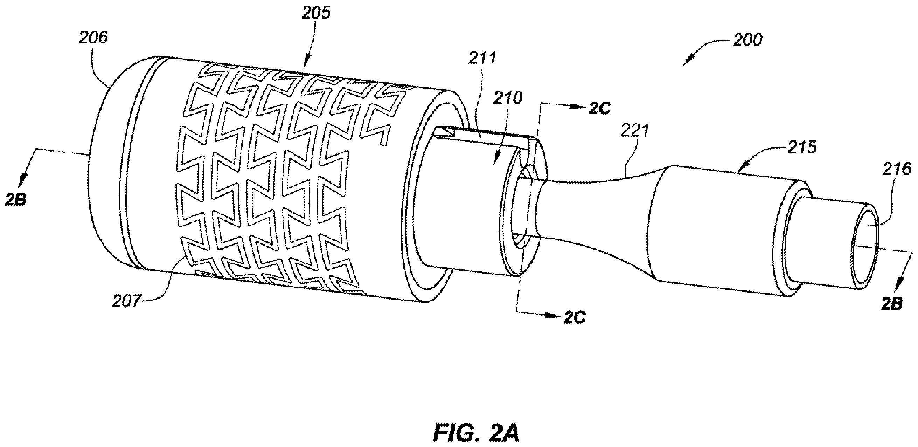

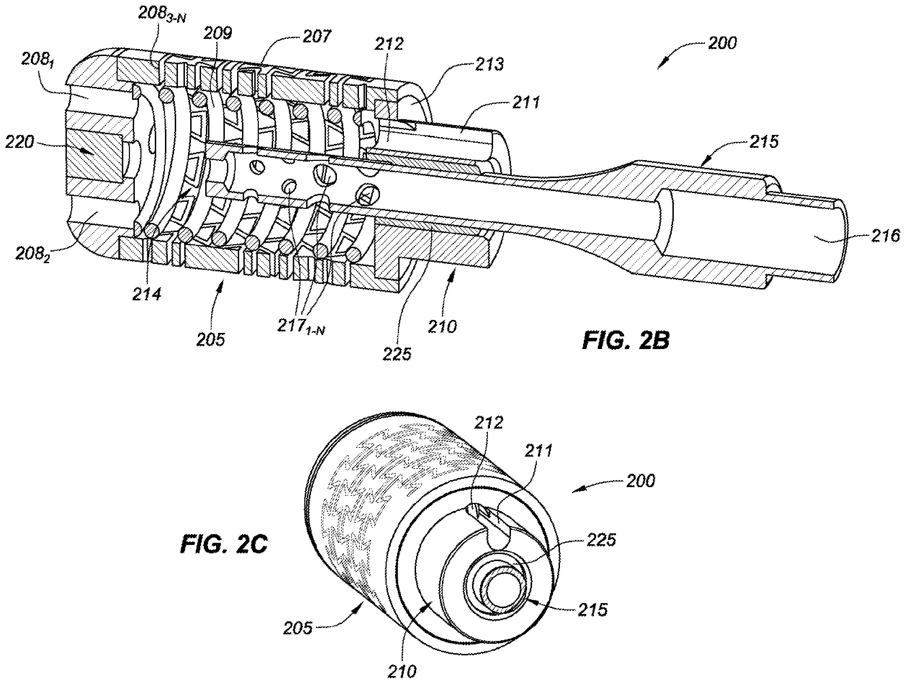

FIG. 2A is an isometric side view of a partial ablation catheter tip assembly 200, FIG. 2B is a cross-sectional side view of the partial ablation catheter tip assembly of FIG. 2A, and FIG. 2C is a cross-sectional, isometric back view of the partial ablation catheter tip assembly of FIG. 2A, consistent with various embodiments of the present disclosure.

Referring to FIGs. 2A-C, the partial ablation catheter tip assembly 200 includes a flex tip 205, that is coupled to a manifold 215 via a tip stem 210. The manifold 215 may be comprised of, for example, a stainless steel alloy, MP35N (a cobalt chrome alloy), titanium alloy, or a composition thereof. The flex tip 205 includes a distal tip 206 and a flexible member portion 207. The flexible member 207 facilitates deformation of the flex tip in response to contact with tissue; more specifically, the flexible member 207 deforms to increase surface contact with target tissue. The increased tissue surface contact improves various diagnostics and therapies (e.g., tissue ablation). After contact with target tissue is complete, a spring 209 returns the flexible member 207 to an un-deformed state. The distal tip 206 may be coupled to the flexible member 207 via an adhesive, weld, etc. Similarly the tip stem 210 may be coupled to the flexible member 207 via an adhesive, weld, etc. A distal end of the manifold 215 extends through the tip stem 210 and is inserted into the flex tip 205. To seal a gap between the tip stem 210 and the manifold 215, a gasket 225 (e.g., o-ring) may be inserted therebetween. In some embodiments, the gasket 225 is a medical-grade thermoplastic polyurethane elastomer. For example, the gasket 225 may be Lubrizol LifeSciences' Pellethane^{®} 2363-90AE TPU, silicone, or another material with similar material characteristics. To further ensure a seal of the gasket 225, and to help couple the gasket 225 between the tip stem 210 and the manifold, adhesive beads may be placed along, for example, an inner and/or outer diameter of the gasket 225, as well as proximal and distal ends thereof. In yet other embodiments, the gasket 225 may be formed of adhesive itself. To facilitate assembly, the gasket 225 may be over-molded onto an outer diameter of the manifold 215 or an inner diameter of the tip stem.

In various embodiments of the present disclosure, to limit the deformation of a structural member, partial ablation catheter tip assembly 200 may be designed to transmit approximately 50% of a force exerted on flex tip 205 through a manifold 215. The manifold 215 transmits the force to a catheter shaft that is coupled to a proximal end of the tip assembly 200. To facilitate large force loads on the manifold 215, the manifold may include a strain relief 221 which limits lateral deflection of the manifold 215 in response to a force exerted on the tip assembly 200 transverse to a longitudinal axis.

Manifold 215 includes an irrigant lumen 216 that delivers irrigant from a distal end of the catheter shaft to a dispersion chamber 214 within the flex tip 205 via manifold apertures 217_{1-N}. The placement of the manifold apertures 217_{1-N} both along a length and circumference of a distal tip of the manifold 215 help facilitate even distribution of irrigant throughout the dispersion chamber 214. Once inside the dispersion chamber 214, the irrigant exits the flex tip 205 via irrigant apertures 208_{1-N}, by virtue of positive pressure therein.

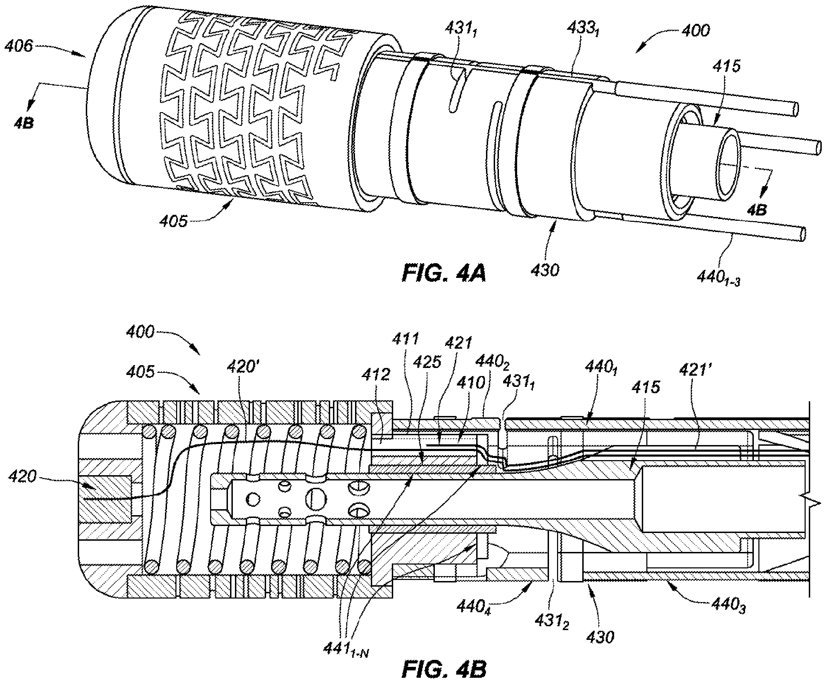

To measure real-time temperature of tissue in contact with a distal tip 206 of the tip assembly 200, it is desirable to position a thermocouple 220 as proximal to the tissue as possible. In the present embodiment, the thermocouple 220 is positioned so that a surface of the thermocouple 220 may be directly, thermally coupled to the tissue. To facilitate desired positioning of the thermocouple, while preventing irrigant from within dispersion chamber 214 from flowing proximally into a structural member 430 (see, e.g., FIG. 4A), thermocouple wires must be ran through the tip stem 210 in such a way as to prevent back-flow. Importantly, as many embodiments of the structural member 430 are coupled with a measurement system that relies upon time-of-flight calculations, ingress of irrigant into the structural member 430 may render the force sensor inoperable or at least inaccurate due to the varying photon speeds through air and liquid. Aspects of the present disclosure are directed to routing a thermocouple wire through tip stem 210 in such a manner as to limit the potential for such ingress. Accordingly, the tip stem 210 of FIGs. 2A-C includes a channel 211 that extends into an outer surface of the tip stem and runs distally, parallel to a longitudinal axis of the tip assembly 200. Upon reaching a radially extending proximal surface 213 of the tip stem 210, the channel 211 intercepts a thermocouple aperture 212 which extends distally through the remaining tip stem 210. The combination of the channel 211 and aperture 212 facilitate routing a thermocouple wire through the tip stem without compromising a seal between an inner diameter of the tip stem 210 and an outer diameter of the manifold 215. To seal the aperture 212 after the thermocouple has been routed there-through, an adhesive (e.g., silicone, epoxy, or other waterproof adhesive) may be packed in and around the thermocouple wire extending through the channel 211 and aperture 212.

In some embodiments, a flexible member 207 of flex tip 205 may comprise a composition including a titanium alloy (or other metal alloy with characteristics including a high tensile strength, e.g., titanium).

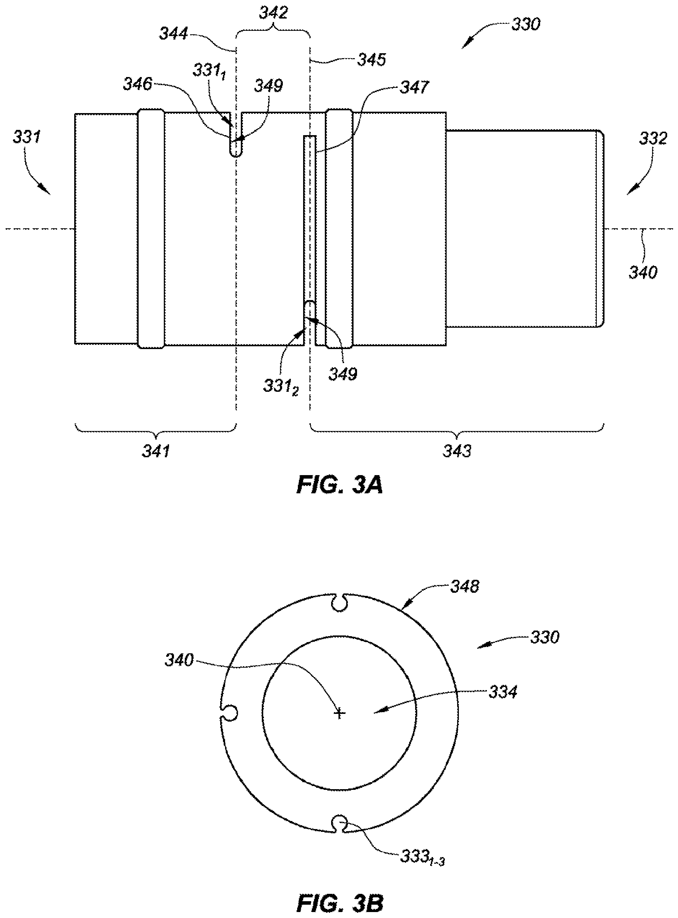

FIG. 3A is a side view of a structural member 330 for a fiber optic force sensing assembly, and FIG. 3B is a front view of the structural member 330 of FIG. 3A, consistent with various embodiments of the present disclosure. Referring to FIGs. 3A and 3B, the structural member 330 of the fiber optic force sensing assembly is designed to house a plurality of fiber optics (see, e.g., FIG. 4A) that extend through grooves 333₁₋₃. In this embodiment, the structural member 330 is divided into a plurality of segments along a longitudinal axis 340 thereof. The plurality of segments including a distal segment 341 extending between a distal end 331 of the structural member 330 and a first flexure portion 331₁, an intermediate segment 342 that extends between the first flexure portion 331₁ and a second flexure portion 331₂, and a proximal segment 343 that extends between the second flexure portion 331₂ and a proximal end 332 of the structural member 330. The segments may be adjacent each other in a serial arrangement along the longitudinal axis 340.

The segments 341, 342, 343 are bridged by flexure portions 331₁₋₂, each flexure portion defining a neutral axes 344 and 345. Each of the neutral axes constitute a location within the respective flexure portions where the stress is zero when subjected to a pure bending moment in any direction.

In some embodiments, adjacent members of the segments may define a plurality of gaps 346 and 347 at the flexure portions 331₁₋₂, each having a separation dimension. It is noted that while the longitudinal separation dimensions of the gaps are depicted as being uniform, the separation dimensions may vary across a given gap, or between gaps. Moreover, the radial dimension of the gaps may also vary (e.g., to compensate for the effects of a moment exerted along a length of the structural member 330).

The structural member 330 may include a plurality of grooves 333₁₋₃ that are formed within an outer surface 348 of the structural member. The grooves 333₁₋₃ may be spaced rotationally equidistant (i.e. spaced 90° apart where there are three grooves) about the longitudinal axis 340 and may be oriented parallel with a longitudinal axis 340 of the structural member 330. Each of the grooves may terminate at a respective one of the gaps 346 and 347 of the flexure portions 331₁₋₂. For example, groove 333₁ may extend along the proximal segment 343 and intermediate segment 342 terminating at the gap 346 at flexure portion 331₁. Other grooves, such as groove 333₂ may extend along the proximal segment 343 terminating at the gap 347 at flexure portion 331₂.

In a fiber optic force sensing assembly, fiber optics may be disposed in the grooves 333₁₋₃, respectively, such that the distal ends of the fiber optics terminate at the gaps 346 and 347 of either flexure portion 331₁₋₂. For example, a fiber optic may extend along groove 333₁, terminating proximate or within the gap 346 at flexure portion 331₁. Likewise, a second fiber optic may extend along the groove 333₂ and terminate proximate or within the gap 347 at flexure portion 331₂. Surfaces 349 of the flexure portions 331₁₋₂, opposite the distal ends of first and second fiber optics, may be coated with a highly reflective material, or third and fourth fiber optics with mirrored surfaces positioned opposite the first and second fiber optics, relative to the gaps 346 and 347. Alternatively, a fiber Bragg grating strain sensor may be implemented.

The gaps 346 and 347 at the flexure portions 331₁₋₂ may be formed so that they extend laterally through a major portion of the structural member 330. Also, the gaps may be oriented to extend substantially normal to a longitudinal axis 340 of the structural member 330, or at an acute angle with respect to the longitudinal axis. In the depicted embodiment, the structural member 330 comprises a hollow cylindrical tube with the flexure portions comprising slots that extend transverse to the longitudinal axis 340 through one side of the hollow cylindrical tube. In many embodiments, the slots extend into an inner diameter 334 of the structural member, and in some cases through the longitudinal axis.

As shown in FIGs. 3A, and 3B, the flexure portions 331₁₋₂ define a semi-circular segment that intercepts the inner diameter 334 of the hollow cylindrical tube. The radial depth of the slots 346 and 347 can be tuned to establish a desired flexibility of the various flexure portions 331₁₋₂. That is, the greater the depth of the flexure portions 331₁₋₂ the more flexible the flexure portions are. The flexure portions 331₁₋₂ may be formed by the various ways available to the artisan, such as but not limited to sawing, laser cutting or electro-discharge machining (EDM). The slots which form the flexure portions 331₁₋₂ may be formed to define non-coincident neutral axes.

When a fiber optic force sensor consistent with the above is assembled, one or more fiber optics are mechanically coupled to structural member 330 via grooves 333₁₋₃. In some embodiments, each of the fiber optics may be communicatively coupled to a Fabry-Perot strain sensor within one of the slots which form the flexure portions 331₁₋₂. The Fabry-Perot strain sensor includes transmitting and reflecting elements on either side of the slots to define an interferometric gap. The free end of the transmitting element may be faced with a semi-reflecting surface, and the free end of the reflecting element may be faced with a reflecting surface.

In some assemblies of a fiber optic force sensor assembly, the fiber optics may be positioned along the grooves 333₁₋₃ (as shown in FIG. 3B) so that the respective Fabry-Perot strain sensor is bridged across one of the flexure portions 331₁₋₂. For example, a fiber optic may be positioned within groove 333₁ so that the Fabry-Perot strain sensor bridges the gap at the flexure portion 331₂ between distal and intermediate segments, 341 and 342, respectively, of structural member 330.

In some embodiments, structural member 330 may comprise a composition including a stainless steel alloy (or other metal alloy with characteristics including a high tensile strength, e.g., titanium).

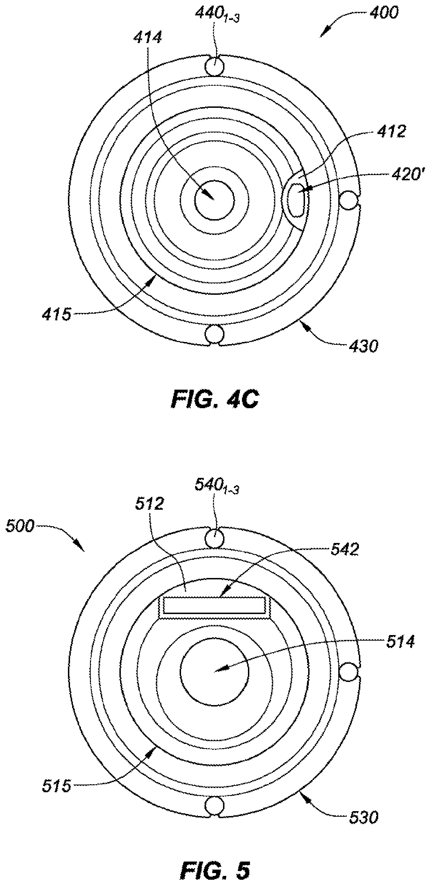

FIG. 4A is an isometric side view of a partial ablation catheter tip assembly 400 including a structural member 430, FIG. 4B is a cross-sectional side view of the partial ablation catheter tip assembly of FIG. 4A, and FIG. 4C is a back view of the partial ablation catheter tip assembly of FIG. 4A, consistent with various embodiments of the present disclosure.

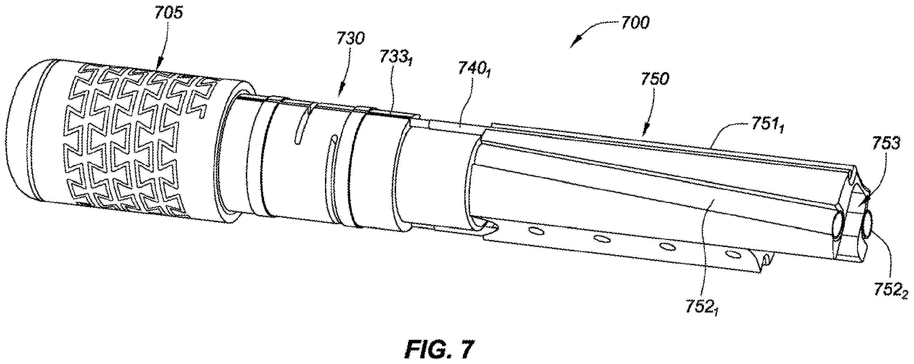

Referring to FIGs. 4A-C, a partial ablation catheter tip assembly 400 includes a structural member 430 which is coupled to a tip assembly 200 (as shown in FIGs. 2A-C). The structural member 430 may be coupled at a distal end to tip stem 410, and at a proximal end to manifold 415. Once the tip assembly 400 is complete, it may be further coupled at a proximal end to a catheter shaft that extends to a catheter handle, or, as discussed in more detail in reference to FIG. 7, the tip assembly 400 may be further coupled to a coupler. The structural member 430 is designed in such a way as to receive forces exerted on the distal tip 406 of the catheter tip assembly 400 and to absorb such force by deflecting and deforming in response thereto. Further, the structural member 430 may be outfitted with a measurement device which facilitates measurement of the deflection/deformation which may be correlated with the force exerted on the distal tip 406 and communicated with the catheter operating clinician. Knowledge of a force exerted on the distal tip 406 of a catheter may be useful for a number of different cardiovascular operations, among other types of operations. For example, during a myocardial tissue ablation therapy it is desirable to know a contact force exerted by the distal tip 406 of the catheter on target tissue, as the time to necrose tissue is based on energy transferred between the catheter and tissue - which is highly dependent upon the extent of tissue contact.

As shown in FIGs. 4A and 4B, the present embodiment utilizes a fiber-optic based measurement system. Fiber-optic cables 440₁₋₄ are coupled to grooves (including groove 433₁) along an outer diameter of structural member 430. Accordingly, a light source may be applied to one or more of the fiber-optic cables and a time-of-flight measurement may be recorded for one or more wave-lengths of light to cross a gap between the fiber-optic pairs in flexure portions 431₁₋₂. In various embodiments, the fiber-optic cables run proximally along a shaft to a catheter handle, which may include processor circuitry or be communicatively coupled to the processor circuitry. The sensed time-of-flight across the flexure portions 431₁₋₂ may be associated with a deflection of the deformable body from a static distance across the flexure portions. During calibration, the structural member 430 may be tested to determine a calibration matrix which associates deformation of the structural member with an applied-force at distal tip 406.

As discussed in more detail in reference to FIGs. 2A-B, manifold 415 is coupled to tip stem 410 via a tube 425 (comprising, for example, Pellethane^{®} 2363-90AE TPU silicone) that prevents ingress of irrigant from flex tip 405, through the interface, and into the structural member 430. To further ensure a proper seal, and coupling of the manifold to the tip stem, adhesive 441_{1-N} (e.g., silicone, epoxy, or other waterproof adhesive) may be applied at various locations of the interface.

In various catheter applications, it may be desirable to place a thermocouple (or other temperature monitoring sensor) as far distal on the catheter as possible to facilitate near real-time temperature measurements; this may be particularly valuable for ablation catheters. Accordingly, the partial ablation catheter tip assembly 400 of FIGs. 4A-C includes a thermocouple 420 positioned in contact with a surface of distal tip 406. However, it can be difficult to seal a joint between a tip stem 410 and manifold 415, while also running a thermocouple wire 420' there through - rendering an embodiment with a thermocouple distal of a structural member 430 difficult to implement. Aspects of the present disclosure are directed to routing a thermocouple wire through a separate sealing point to improve overall sealing of a structural member 430 from irrigant being delivered to the flex tip 405. As shown in FIGs. 4B-C, thermocouple wire 420' extends through a channel 411 and aperture 412 in tip stem 410 and into a dispersion chamber 214 (see, FIG. 2B) of the flex tip 405. The aperture 412 and/or channel 411 may then be packed with a sealant, to prevent ingress of irrigant into the structural member 430. By dissociating the seal between the tip stem 410 and manifold 415, and the tip stem 410 and thermocouple wire 420', the overall sealing efficacy between the dispersion chamber and structural member are greatly improved. The thermocouple wire 420' further travels distally through an inner diameter of the structural member and into a lumen of the catheter shaft that extends to the catheter handle.

As shown in FIG. 4B, tip stem 410 is seated to and coupled with an inner diameter of structural member 430 at a proximal end, and is seated to and coupled with an inner diameter of flex tip 405 at a distal end. The tip stem structurally transmits a force exerted on the flex tip 405 to both structural member 430 and manifold 415. By diverting a portion of the force exerted to the manifold, the tip assembly 400 as a whole exhibits improved stiffness especially in regard to lateral deflection. In some embodiments, the manifold may absorb up to 50%, or more, of the force exerted on the distal tip. Moreover, in some specific embodiments, the structural member 430 has an increased inner diameter and outer diameter, with pivot points of the flexure portions 431 being extended radially outward. Accordingly, the structural member 430 is stiffer and receives less of the force exerted on the distal tip 406, resulting in the structural member 430 experiencing less deflection and being less susceptible to plastic deformation during delivery of the catheter to a therapy site (e.g., via introducer sheath). Moreover, by transmitting force onto the manifold 415, the structural member 430 may be tuned to improve the ratio of lateral-to-axial deflection.

In some specific embodiments, as shown in Fig. 4B, partial ablation catheter tip assembly 400 may further include a second thermocouple 421 which is positioned within channel 411 and/or aperture 412 of tip stem 410. A lead wire 421' and/or flexible circuitry may be communicatively coupled to the second thermocouple and extend proximally to a catheter handle of the catheter. Alternatively the second thermocouple 421 may be communicatively coupled to the thermocouple wire 420' which is extending proximally from thermocouple 420 positioned at a distal tip 406 of the assembly 400. As this second thermocouple 421 is placed in close proximity to deformable body 430, signals therefrom may be used by processor circuitry to conduct temperature compensation of the force readings sensed by the measurement system. The deformable body, in response to rapid temperature changes associated with the energy released by the ablation tip, is prone to expansion and contraction, which may result in significant force measurement variation by the measurement system. The processor circuitry may use signals from the thermocouple 420 in the tip stem to identify the likelihood of error in the force measurement reading and to compensate therefore. For example, the processor circuitry may utilize an algorithm, calibration matrix, etc. to compensate for the temperature-induced error. Aspects of such temperature compensation may be calibrated and tested at the factory to account for unit-to-unit variation in the deformable body.

Fig. 4C further shows grooves (see, e.g., FIG. 4A) that extend into an outer surface of structural member 430, and through which fiber optics 440₁₋₃ extend to their respective flexure portions. Manifold 415 is seated to and coupled with an inner diameter of a proximal end of structural member 430. An inner lumen of the manifold 415 extends along a longitudinal axis of the manifold to deliver irrigant to dispersion chamber 414 within a flexible tip of the catheter tip assembly 400.

While various embodiments of the present disclosure are discussed in reference to an ablation catheter, it is to be understood that a catheter consistent with the present disclosure may implement various different types of end effectors - e.g., mapping electrodes or ablation electrodes, such as are known in the art for diagnosis or treatment of a vessel or organ may be utilized with the present invention. For example, the catheter tip assembly 400 may be configured as an electrophysiology catheter for performing cardiac mapping and ablation. In other embodiments, the catheter tip assembly 400 may be configured to deliver drugs or bioactive agents to a vessel or organ wall or to perform minimally invasive procedures such as, for example, cryo-ablation.

FIG. 5 is a back view of an alternative, partial ablation catheter tip assembly 500, consistent with various embodiments of the present disclosure. A proximal end of a manifold 515 is coupled to an inner diameter of structural member 530. The manifold may include a channel that extends into an outer diameter of the manifold and extends distally to a tip stem with a corresponding channel and aperture 512 which facilitates routing of flexible circuitry 542 through the manifold and tip stem, and into a dispersion chamber 514 of the flex tip before electrically coupling to a thermocouple, for example. In yet other embodiments, the flexible circuitry 542 may be electrically coupled to, for example, but not necessarily limited to a thermocouple, spot electrodes, flow sensors, a radio-frequency signal emitter, etc. To seal the aperture 512 after the flexible circuitry 542 has been routed there-through, an adhesive may be packed in and around the flexible circuitry extending through the aperture 512. The adhesive seals the aperture 512 from irrigant ingress into the structural member 530. By dissociating the seal between the tip stem and manifold 515, and the tip stem and flexible circuitry 542, the overall sealing efficacy between the dispersion chamber and structural member are greatly improved.

Fig. 5 further shows grooves that extend into an outer surface of structural member 530, and through which fiber optics 540₁₋₃ extend to their respective flexure portions.

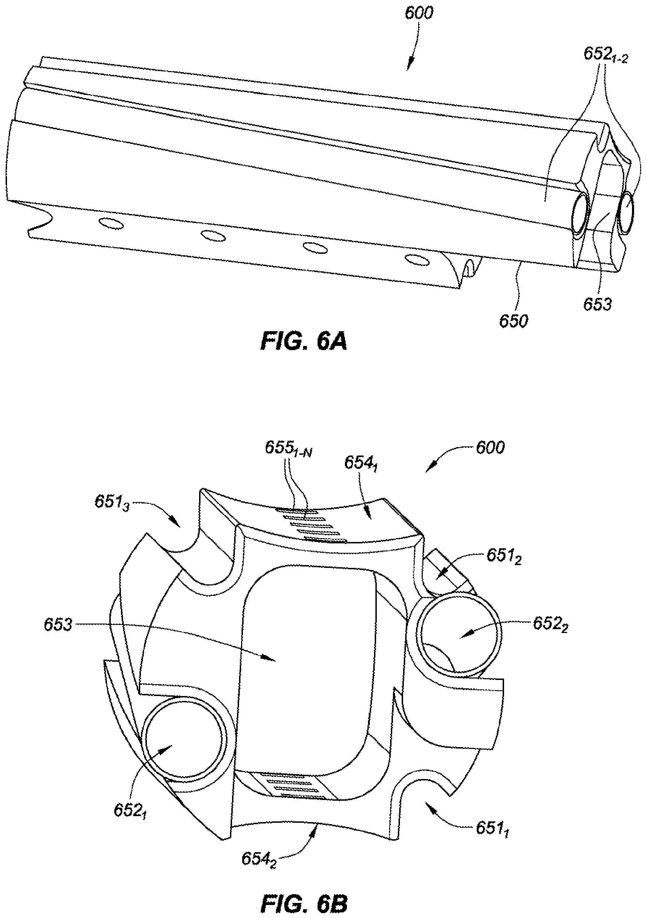

FIG. 6A is an isometric side view of a sensor coupler assembly 600, and FIG. 6B is an isometric front view of the sensor coupler assembly of FIG. 6A, consistent with various embodiments of the present disclosure. As shown in FIGs. 6A-B, the sensor coupler assembly 600 includes a center lumen 653 which facilitates, for example, running various wires and irrigant lumens distally to a catheter tip. The coupler body 650 facilitates the coupling of an ablation catheter tip assembly 400 (see, e.g., FIG. 4A) to a catheter shaft. In the present embodiment, the coupler has been adapted to facilitate the mounting of magnetic localization coils 652₁₋₂ to grooves extending into an outer diameter of the coupler body 650. When located within a controlled magnetic field, the pair of magnetic localization coils 652₁₋₂ produce an electrical signal that is indicative of the six degrees of freedom that the catheter tip has within space. In some advanced embodiments, the location of the catheter tip may be associated with a position within a patient's anatomy and displayed for the clinician to reference during a procedure.

Fiber optic channels 651₁₋₃ extend longitudinally along an outer diameter of the coupler body 650, and align with grooves (see, e.g., FIG. 7 - groove 733₁ ) on the structural member 730. Inverted radiuses 654₁₋₂ in an outer diameter of the coupler body 650 facilitate the flow of adhesive in and around the coupler when being coupled with an external housing. To further facilitate adhesive flow, flow apertures 655_{1-N} extend radially into a center lumen 653 of coupler body 650, and some of the adhesive sandwiched between the inverted radiuses 654₁₋₂ and an inner diameter of the external housing may escape into the flow apertures 655_{1-N}. The inverted radiuses 654₁₋₂ may also house, for example, ring electrode wires extending between ring electrodes near a distal tip of the catheter and a catheter handle.

FIG. 7 is an isometric side view of a partial ablation catheter tip assembly 700 including a coupler body 750, consistent with various embodiments of the present disclosure. A distal end of the coupler body 750 is mounted to a proximal end of structural member 730 (which is mounted to a tip stem and flex tip 705). Fig. 7 shows one or more fiber optics 740₁ extending through channels 751₁ (not all of fiber optics and channels are shown in Fig. 7) of the coupler body 750 and grooves (including groove 733₁) of the structural member 730. The relatively proximal placement of the coupler body 750 relative to the flex tip 705 allows for accurate magnetic localization of the catheter tip via magnetic localization coils 752₁₋₂. A central lumen 753 facilitates an irrigant lumen extending there through and into fluid communication with a manifold that extends through an inner diameter of the structural member 730 and flex tip 705.

Further, it is referred to United States provisional application no. 62/331,292, filed 3 May 2016, United States application no. 15/585,859, filed 3 May 2017, and international application no. PCT/US17/30828, filed 3 May 2017.

Although several embodiments have been described above with a certain degree of particularity, those skilled in the art could make numerous alterations to the disclosed embodiments without departing from the scope of the present disclosure. It is intended that all matter contained in the above description or shown in the accompanying drawings shall be interpreted as illustrative only and not limiting. Changes in detail or structure may be made without departing from the present teachings. The foregoing description and following claims are intended to cover all such modifications and variations.

Various embodiments are described herein of various apparatuses, systems, and methods. Numerous specific details are set forth to provide a thorough understanding of the overall structure, function, manufacture, and use of the embodiments as described in the specification and illustrated in the accompanying drawings. It will be understood by those skilled in the art, however, that the embodiments may be practiced without such specific details. In other instances, well-known operations, components, and elements have not been described in detail so as not to obscure the embodiments described in the specification. Those of ordinary skill in the art will understand that the embodiments described and illustrated herein are non-limiting examples, and thus it can be appreciated that the specific structural and functional details disclosed herein may be representative and do not necessarily limit the scope of the embodiments, the scope of which is defined solely by the appended claims.

Reference throughout the specification to "various embodiments," "some embodiments," "one embodiment," "an embodiment," or the like, means that a particular feature, structure, or characteristic described in connection with the embodiment is included in at least one embodiment. Thus, appearances of the phrases "in various embodiments," "in some embodiments," "in one embodiment," "in an embodiment," or the like, in places throughout the specification are not necessarily all referring to the same embodiment. Furthermore, the particular features, structures, or characteristics may be combined in any suitable manner in one or more embodiments. Thus, the particular features, structures, or characteristics illustrated or described in connection with one embodiment may be combined, in whole or in part, with the features structures, or characteristics of one or more other embodiments without limitation.

It will be appreciated that the terms "proximal" and "distal" may be used throughout the specification with reference to a clinician manipulating one end of an instrument used to treat a patient. The term "proximal" refers to the portion of the instrument closest to the clinician and the term "distal" refers to the portion located furthest from the clinician. It will be further appreciated that for conciseness and clarity, spatial terms such as "vertical," "horizontal," "up," and "down" may be used herein with respect to the illustrated embodiments. However, surgical instruments may be used in many orientations and positions, and these terms are not intended to be limiting and absolute.

## Claims

1. A force-sensing catheter system comprising
a catheter tip (205; 405; 705),
a deformable body (330; 430; 730) coupled to the catheter tip (205; 405; 705) and including a lumen that extends along a longitudinal axis of the force-sensing catheter system, the deformable body is configured and arranged to deform in response to a force exerted on the catheter tip (205; 405; 705), and
a manifold (215; 415) extending through the lumen of the deformable body (330; 430; 730), wherein the manifold (215; 415) is coupled to the catheter tip (205; 405; 705) and a proximal end of the deformable body (330; 430; 730),
wherein the manifold (215; 415) is configured and arranged to deliver irrigant to the distal tip (205; 405; 705), and
**characterised in that**
the manifold (215; 415) is further configured and arranged to transmit a portion of the force exerted on the catheter tip (205; 405; 705), proximally, to the proximal end of the deformable body (330; 430; 730).

2. The force-sensing catheter system of claim 1, wherein the manifold (215; 415) and deformable body (330; 430; 730) are further configured and arranged to emulate a desired lateral-to-axial compliance ratio of the force-sensing catheter system by transmitting a portion of the force exerted on the catheter tip (205; 405; 705) through the manifold (215; 415).

3. The force-sensing catheter system of claim 1 or 2, further including a hollow tip stem (210; 410) including an inner and outer diameter, and an aperture (212; 412) that extends through a length of the tip stem (210; 410), the outer diameter of the tip stem (210; 410) is coupled to the catheter tip (205; 405; 705) and the deformable body (330; 430; 730), and the manifold (215; 415) is coupled to and extends through the inner diameter of the tip stem (210; 410).

4. The force-sensing catheter system of claim 3, further including a dispersion chamber (214) within the catheter tip (205; 405; 705), and a seal coupled between the outer diameter of the manifold (215; 415) and the inner diameter of the tip stem (210; 410), the seal configured and arranged to hermetically seal the dispersion chamber (214) from the deformable body (330; 430; 730).

5. The force-sensing catheter system of claim 4, wherein the seal is a thermoplastic polyurethane elastomer that circumferentially extends around an outer diameter of the manifold (215; 415).

6. The force-sensing catheter system of claim 3, 4 or 5, further including a thermocouple (220; 420) coupled near a distal end of the catheter tip (205; 405; 705), and a wire (420') or flexible electronic circuit (542) communicatively coupled with the thermocouple (210; 410) and extending proximally through the aperture of the tip stem, the aperture of the tip stem (220; 420) is configured and arranged to facilitate hermetically sealing the aperture (212; 412) with the wire (420') or flexible electronic circuit (542) extending there through.

7. The force-sensing catheter system of claim 6, further including a second thermocouple (421) coupled within the aperture (412) of the tip stem (410), the second thermocouple (421) configured and arranged to measure a temperature in proximity to the deformable body (405), the measured temperature indicative of temperature induced expansion and contraction of the deformable body (405).

8. The force-sensing catheter system of any one of claims 1 to 7, further including
a measurement system coupled to the deformable body, the measurement system including three or more sensing elements (440), the sensing elements (440) configured and arranged to detect the deformation of the deformable body (330; 430; 730), in response to the force exerted on the catheter tip (205; 405; 705), and transmit a signal indicative of the deformation;
processor circuitry communicatively coupled to the measurement system, and configured and arranged to receive the signal from each of the force sensing elements (440), indicative of the deformation, and to determine a magnitude of the force exerted on the catheter.

9. The force-sensing catheter system of claim 8, wherein the sensing elements (440) are optical fibers, and the signal received from the optical fibers are photons,
and/or
wherein the sensing elements are circumferentially distributed about the longitudinal axis of the deformable body (330; 430; 730).

10. The force-sensing catheter system of claim 8 or 9, wherein the processing circuitry is further configured and arranged to determine a time-of-flight of photons across the deformable body (330; 430; 730) and to associate time-of-flight with the force exerted on the catheter tip (205; 405; 705).

11. The force-sensing catheter system of any one of claims 8 to 10, further including a display communicatively coupled to the processor circuitry, wherein the processor circuitry is further configured and arranged to transmit data packets to the display indicative of the force exerted on the catheter tip (205; 405; 705), and the display is configured and arranged to communicate the force to a clinician.

12. The force-sensing catheter system of claims 1 to 11, wherein the catheter tip (205; 405; 705) is configured and arranged to flex in response to contact with tissue and to thereby improve tissue contact therewith, and return to an undeformed state after contact with tissue has ceased.

13. The force-sensing catheter system of any one of claims 1 to 12, further including a sensor coupler assembly (600; 700) coupled to the proximal end of the deformable body (330; 430; 730) and the manifold (215; 415), a catheter shaft coupled to a proximal end of the sensor coupler assembly (600; 700), and a handle coupled to a proximal end of the catheter shaft, the sensor coupler assembly (600; 700) including
a coupler body including one or more channels (651; 751) circumferentially distributed along a length of the coupler body, and a center lumen (653; 753) that extends along a longitudinal axis of the coupler body, and
two or more magnetic localization coils (652; 752) mechanically coupled to the one or more channels of the coupler body, and in a nonparallel orientation relative to one another, the two or more magnetic localization coils configured and arranged to transmit an electrical signal indicative of the six degrees of freedom that the catheter tip has within a controlled magnetic field.

14. The force sensor catheter system of any one of claims 1 to 13, wherein
the distal tip is configured and arranged to deliver energy to contacted tissue, and
the manifold (215; 415) is mechanically coupled to the proximal end of the distal tip (205; 405; 705), and the manifold is configured and arranged to limit deformation of the deformable body in response to the force exerted on the distal tip by absorbing a portion of the exerted force.

## Patentansprüche

1. Kraftmessendes Kathetersystem, mit
einer Katheterspitze (205; 405; 705),
einem verformbaren Körper (330; 430; 730), der an die Katheterspitze (205; 405; 705) gekoppelt ist und ein Lumen aufweist, das sich entlang einer Längsachse des kraftmessenden Kathetersystems erstreckt, bei dem der verformbare Körper konfiguriert und angeordnet ist, sich in Antwort auf eine Kraft, die auf die Katheterspitze (205; 405; 705) ausgeübt wird, zu verformen, und
einem Verteilerrohr (215; 415), das sich durch das Lumen des verformbaren Körpers (330; 430; 730) erstreckt, bei dem das Verteilerrohr (215; 415) an die Katheterspitze (205; 405; 705) und an ein proximales Endes des verformbaren Körpers (330; 430; 730) gekoppelt ist,
bei dem das Verteilerrohr (215; 415) dazu konfiguriert und angeordnet ist, ein Irrigant an die distale Spitze (205; 405; 705) zuzuführen, und
**dadurch gekennzeichnet, dass**
das Verteilerrohr (215; 415) weiter dazu konfiguriert und angeordnet ist, einen Teil der Kraft, die auf die Katheterspitze (205; 405; 705) ausgeübt wird, proximal dem proximalen Ende des verformbaren Körpers (330; 430; 730) zu übertragen.

2. Kraftmessendes Kathetersystem nach Anspruch 1, bei dem das Verteilerrohr (215; 415) und der verformbare Körper (330; 430; 730) weiter dazu konfiguriert und angeordnet sind, ein gewünschtes Verhältnis von seitlicher zu axialer Konformität des kraftmessenden Kathetersystems durch Übertragen eines Teils der Kraft, die auf die Katheterspitze (205; 405; 705) aufgebracht wird, durch das Verteilerrohr (215; 415) zu emulieren.

3. Kraftmessendes Kathetersystem nach Anspruch 1 oder 2, ferner mit einem hohlen Spitzenbolzen (210; 410), der einen inneren und einen äußeren Durchmesser aufweist, und einer Öffnung (212; 412), die sich durch eine Länge des Spitzenbolzens (210; 410) erstreckt, bei dem der Außendurchmesser des Spitzenbolzens (210; 410) an die Katheterspitze (205; 405; 705) und den verformbaren Körper (330; 430; 730) gekoppelt ist, und das Verteilerrohr (215; 415) an den Innendurchmesser des Spitzenbolzens (210; 410) gekoppelt ist und sich durch diesen erstreckt.

4. Kraftmessendes Kathetersystem nach Anspruch 3, ferner mit einer Dispersionskammer (214) innerhalb der Katheterspitze (205; 405; 705) und einer Dichtung, die zwischen dem Außendurchmesser des Verteilerrohrs (215; 415) und dem Innendurchmesser des Spitzenbolzens (210; 410) gekoppelt ist, bei dem die Dichtung dazu konfiguriert und angeordnet ist, hermetisch die Dispersionskammer (214) gegenüber dem verformbaren Körper (330; 430; 730) abzudichten.

5. Kraftmessendes Kathetersystem nach Anspruch 4, bei dem die Dichtung ein thermoplastisches Polyurethan-Elastomer ist, das sich umfänglich um einen Außendurchmesser des Verteilerrohrs (215; 415) erstreckt.

6. Kraftmessendes Kathetersystem nach Anspruch 3, 4, oder 5 ferner mit einem Temperaturmessgeber (220; 420), der nahe einem distalen Ende der Katheterspitze (205; 405; 705) gekoppelt ist, und einem Draht (420') oder einer flexiblen elektronischen Schaltung (542), die kommunikativ mit dem Temperaturmessgeber (210; 410) gekoppelt ist und sich proximal durch die Öffnung des Spitzenbolzens (210; 410) erstreckt, bei dem die Öffnung des Spitzenbolzens (220; 420) dazu konfiguriert und angeordnet ist, ein hermetisches Abdichten der Öffnung (212; 412) mit dem Draht (420') oder der flexiblen elektronischen Schaltung (542) sich dort hindurcherstreckend zu ermöglichen.

7. Kraftmessendes Kathetersystem nach Anspruch 6, ferner mit einem zweiten Temperaturmessgeber (421), der innerhalb der Öffnung (412) des Spitzenbolzens (410) gekoppelt ist, bei dem der zweite Temperaturmessgeber (421) dazu konfiguriert und angeordnet ist, eine Temperatur in der Umgebung des verformbaren Körpers (405) zu messen, bei dem die gemessene Temperatur eine Temperatur anzeigt, die durch Expansion und Kontraktion des verformbaren Körpers (405) induziert wird.

8. Kraftmessendes Kathetersystem nach einem der Ansprüche 1 bis 7, ferner mit
einem Messsystem, das an den verformbaren Körper gekoppelt ist, bei dem das Messsystem drei oder mehr Erfassungselemente (440) aufweist, und die Erfassungselemente (440) dazu konfiguriert und angeordnet sind, die Verformung des verformbaren Körpers (330; 430; 730) in Antwort auf die Kraft, die auf die Katheterspitze (205; 405; 705) ausgeübt wird, zu erfassen und ein Signal zu übertragen, das die Verformung anzeigt,
einer Prozessorschaltung, die kommunikativ mit dem Messsystem gekoppelt ist, und dazu konfiguriert und angeordnet ist, das Signal von jedem der Krafterfassungselemente (440) zu empfangen, das die Verformung anzeigt, und eine Größe der Kraft zu bestimmen, die auf den Katheter ausgeübt wird.

9. Kraftmessendes Kathetersystem nach Anspruch 8, bei dem die Erfassungselemente (440) optische Fasern sind, und die Signale, die von den optischen Fasern empfangen werden, Photonen sind,
und/oder
bei dem die Erfassungselemente umfänglich um die Längsachse des verformbaren Körpers (330; 430; 730) verteilt sind.

10. Kraftmessendes Kathetersystem nach Anspruch 8 oder 9, bei dem die Prozessorschaltung ferner dazu konfiguriert und angeordnet ist, eine Flugzeit von Photonen durch den verformbaren Körper (330; 430; 730) zu bestimmen und die Flugzeit mit der auf die Katheterspitze (205; 405; 705) ausgeübten Kraft zu verknüpfen.

11. Kraftmessendes Kathetersystem nach einem der Ansprüche 8 bis 10, ferner mit einer Anzeige, die kommunikativ mit der Prozessorschaltung gekoppelt ist, bei dem die Prozessorschaltung ferner dazu konfiguriert und angeordnet ist, Datenpakete an die Anzeige zu übertragen, die die Kraft anzeigen, die auf die Katheterspitze (205; 405; 705) ausgeübt wird, und die Anzeige dazu konfiguriert und angeordnet sind, die Kraft an einen Mediziner mitzuteilen.

12. Kraftmessendes Kathetersystem nach einem der Ansprüche 1 bis 11, bei dem die Katheterspitze (205; 405; 705) dazu konfiguriert und angeordnet ist, sich in Kontakt mit dem Gewebe zu biegen und dabei den Gewebekontakt damit zu verbessern, und zu einem nicht verformten Zustand zurückzukehren, nachdem der Kontakt mit dem Gewebe beendet wurde.

13. Kraftmessendes Kathetersystem nach einem der Ansprüche 1 bis 12, ferner mit einer Sensorkoppelbaugruppe (600; 700), die an das proximale Ende des verformbaren Körpers (330; 430; 730) und das Verteilerrohr (215; 415) gekoppelt ist, einem Katheterschaft, der an ein proximales Ende der Sensorkoppelbaugruppe (600; 700) gekoppelt ist, und einem Handgriff, der an ein proximales Ende des Katheterschaftes gekoppelt ist, bei dem die Sensorkoppelbaugruppe (600; 700)
einen Koppelkörper, der einen oder mehrere Kanäle (651; 751), die umfänglich entlang einer Länge des Koppelkörpers verteilt sind, und ein mittleres Lumen (653; 753) aufweist, das sich entlang einer Längsachse des Koppelkörpers erstreckt, und
zwei oder mehr Magnetspulen zur Lokalisierung (652; 752) aufweist, die mechanisch an den einen oder mehreren Kanälen des Koppelkörpers und in einer nicht-parallelen Orientierung relativ zueinander gekoppelt sind, bei dem die zwei oder mehreren Magnetspulen zur Lokalisierung derart konfiguriert und angeordnet sind, dass sie ein elektrisches Signal übertragen, das die sechs Freiheitsgrade anzeigt, die die Katheterspitze innerhalb eines gesteuerten Magnetfelds aufweist.

14. Kraftmessendes Kathetersystem nach einem der Ansprüche 1 bis 13, bei dem
die distale Spitze dazu konfiguriert und angeordnet ist, eine Energie dem berührten Gewebe zuzuführen, und
das Verteilerrohr (215; 415) an das proximale Ende der distalen Spitze (205; 405; 705) mechanisch gekoppelt ist, und das Verteilerrohr dazu konfiguriert und angeordnet ist, die Verformung des verformbaren Körpers in Antwort auf die Kraft, die auf die distale Spitze ausgeübt wird, durch Absorbieren eines Teils der ausgeübten Kraft zu beschränken.

## Revendications

1. Système de cathéter à détection de force comprenant
une pointe de cathéter (205 ; 405 ; 705),
un corps déformable (330 ; 430 ; 730) couplé à la pointe de cathéter (205 ; 405 ; 705) et comprenant une lumière qui s'étend le long d'un axe longitudinal du système de cathéter à détection de force, le corps déformable est configuré et agencé pour se déformer en réponse à une force exercée sur la pointe de cathéter (205 ; 405 ; 705), et
un collecteur (215 ; 415) s'étendant à travers la lumière du corps déformable (330 ; 430 ; 730), dans lequel le collecteur (215 ; 415) est couplé à la pointe de cathéter (205 ; 405 ; 705) et à une extrémité proximale du corps déformable (330 ; 430 ; 730),
dans lequel le collecteur (215 ; 415) est configuré et agencé pour délivrer un irrigant à la pointe distale (205 ; 405 ; 705), et
**caractérisé en ce que**
le collecteur (215 ; 415) est en outre configuré et agencé pour transmettre une partie de la force exercée sur la pointe de cathéter (205 ; 405 ; 705), de manière proximale, à l'extrémité proximale du corps déformable (330 ; 430 ; 730).

2. Système de cathéter à détection de force selon la revendication 1, dans lequel le collecteur (215 ; 415) et le corps déformable (330 ; 430 ; 730) sont en outre configurés et agencés pour émuler un rapport de compliance latéral/axial souhaité du système de cathéter à détection de force en transmettant une partie de la force exercée sur la pointe de cathéter (205 ; 405 ; 705) à travers le collecteur (215 ; 415).

3. Système de cathéter à détection de force selon la revendication 1 ou 2, comprenant en outre une tige de pointe creuse (210; 410) comprenant un diamètre intérieur et extérieur, et une ouverture (212 ; 412) qui s'étend à travers une longueur de la tige de pointe (210 ; 410), le diamètre extérieur de la tige de pointe (210 ; 410) est couplé à la pointe de cathéter (205 ; 405 ; 705) et au corps déformable (330 ; 430 ; 730), et le collecteur (215 ; 415) est couplé au diamètre intérieur de la tige de pointe (210 ; 410) et s'étend à travers celui-ci.

4. Système de cathéter de détection de force selon la revendication 3, comprenant en outre une chambre de dispersion (214) à l'intérieur de la pointe de cathéter (205 ; 405 ; 705), et un joint couplé entre le diamètre extérieur du collecteur (215 ; 415) et le diamètre intérieur de la tige de pointe (210 ; 410), le joint étant configuré et agencé pour sceller hermétiquement la chambre de dispersion (214) par rapport au corps déformable (330 ; 430 ; 730).

5. Système de cathéter de détection de force selon la revendication 4, dans lequel le joint est un élastomère de polyuréthane thermoplastique qui s'étend circonférentiellement autour d'un diamètre extérieur du collecteur (215 ; 415).

6. Système de cathéter de détection de force selon la revendication 3, 4 ou 5, comprenant en outre un thermocouple (220 ; 420) couplé près d'une extrémité distale de la pointe de cathéter (205 ; 405 ; 705), et un fil (420') ou un circuit électronique flexible (542) couplé de manière communicative avec le thermocouple (210 ; 410) et s'étendant de manière proximale à travers l'ouverture de la tige de pointe, l'ouverture de la tige de pointe (220 ; 420) est configurée et agencée pour faciliter la fermeture hermétique de l'ouverture (212 ; 412) avec le fil (420') ou le circuit électronique flexible (542) s'étendant à travers celle-ci.

7. Système de cathéter de détection de force selon la revendication 6, comprenant en outre un second thermocouple (421) couplé à l'intérieur de l'ouverture (412) de la tige de pointe (410), le second thermocouple (421) étant configuré et agencé pour mesurer une température à proximité du corps déformable (405), la température mesurée indiquant la dilatation et la contraction induites par la température du corps déformable (405).

8. Système de cathéter à détection de force selon l'une quelconque des revendications 1 à 7, comprenant en outre
un système de mesure couplé au corps déformable, le système de mesure comprenant trois éléments de détection ou plus (440), les éléments de détection (440) étant configurés et agencés pour détecter la déformation du corps déformable (330 ; 430 ; 730), en réponse à la force exercée sur la pointe de cathéter (205 ; 405 ; 705), et transmettre un signal indicatif de la déformation ;
un circuit de processeur couplé de manière communicative au système de mesure, et configuré et agencé pour recevoir le signal de chacun des éléments de détection de force (440), indiquant la déformation, et pour déterminer une magnitude de la force exercée sur le cathéter.

9. Système de cathéter à détection de force selon la revendication 8, dans lequel les éléments de détection (440) sont des fibres optiques, et le signal reçu des fibres optiques sont des photons,
et/ou
dans lequel les éléments de détection sont répartis circonférentiellement autour de l'axe longitudinal du corps déformable (330 ; 430 ; 730).

10. Système de cathéter à détection de force selon la revendication 8 ou 9, dans lequel le circuit de traitement est en outre configuré et agencé pour déterminer un temps de vol des photons à travers le corps déformable (330 ; 430 ; 730) et pour associer le temps de vol à la force exercée sur la pointe de cathéter (205 ; 405 ; 705).

11. Système de cathéter à détection de force selon l'une quelconque des revendications 8 à 10, comprenant en outre un affichage couplé de manière communicative au circuit de processeur, dans lequel le circuit de processeur est en outre configuré et agencé pour transmettre des paquets de données à l'affichage indiquant la force exercée sur la pointe de cathéter (205 ; 405 ; 705), et l'affichage est configuré et agencé pour communiquer la force à un clinicien.

12. Système de cathéter à détection de force selon les revendications 1 à 11, dans lequel la pointe de cathéter (205 ; 405 ; 705) est configurée et agencée pour fléchir en réponse au contact avec le tissu et pour améliorer ainsi le contact du tissu avec celui-ci, et revenir à un état non déformé après que le contact avec le tissu a cessé.

13. Système de cathéter de détection de force selon l'une quelconque des revendications 1 à 12, comprenant en outre un ensemble de coupleur de capteur (600 ; 700) couplé à l'extrémité proximale du corps déformable (330 ; 430 ; 730) et au collecteur (215; 415), un corps de cathéter couplé à une extrémité proximale de l'ensemble de coupleur de capteur (600; 700), et une poignée couplée à une extrémité proximale du corps de cathéter, l'ensemble de coupleur de capteur (600 ; 700) comprenant
un corps de coupleur comprenant un ou plusieurs canaux (651 ; 751) distribués de manière circonférentielle le long d'une longueur du corps de coupleur, et une lumière centrale (653 ; 753) qui s'étend le long d'un axe longitudinal du corps du coupleur, et
deux ou plusieurs bobines de localisation magnétique (652 ; 752) couplées mécaniquement audit ou auxdits canaux du corps de coupleur, et dans une orientation non parallèle l'une par rapport à l'autre, les deux ou plusieurs bobines de localisation magnétique étant configurées et agencées pour transmettre un signal électrique indiquant les six degrés de liberté que la pointe de cathéter possède dans un champ magnétique contrôlé.

14. Système de cathéter à capteur de force selon l'une quelconque des revendications 1 à 13, dans lequel
la pointe distale est configurée et agencée pour délivrer de l'énergie au tissu contacté, et
le collecteur (215 ; 415) est couplé mécaniquement à l'extrémité proximale de la pointe distale (205 ; 405 ; 705), et le collecteur est configuré et agencé pour limiter la déformation du corps déformable en réponse à la force exercée sur la pointe distale en absorbant une partie de la force exercée.
